# EUROPEAN PATENT APPLICATION

(11) **EP 1 875 939 A1**
(43) Date of publication of application: **09.01.2008**
(21) Application number: 07111938.2
(22) Date of filing: 06.07.2007
(51) Int. Cl.: A61M 25/00

(54) **Packaging assembly for a catheter**

(30) Priority: 07.07.2006 US 483325
(71) Applicant: Medtronic Vascular, Inc., Santa Rosa, California 95403 (US)
(72) Inventor: Duffy, Niall, County Galway (IE); Farrell, Thomas, Medtronic Vascular Galway, Galway (IE); Smedley, James, County Galway (IE); Treacy, Kevin, County Galway (IE)
(74) Representative: McKeown, Yvonne Mary

(57) **Abstract**

An assembly (100) for packaging a catheter (20) includes flexible tubing (102) capable of being coiled, and tubular retainer (106) having a passageway extending therethrough. The tubular retainer includes a distal section for coupling to a proximal end (107) of the tubing. A proximal end of the tubular retainer includes an opening for receiving a luer (26) of the catheter assembly. A keyway (108) is provided at a proximal end (126) of the tubular retainer, the keyway being adapted to receive an inflation port (28) of the luer. The keyway may include a snap point (106a) at a proximal end thereof, wherein the snap point is dimensioned such that a force is required to pass the inflation port past the snap point, thereby resulting in a snap fit. The keyway (108c) may include an entry portion (128c) beginning at the proximal end of the tubular retainer (106c), and a nose portion (130c) disposed distal of the entry portion, with a snap point (134) disposed therebetween. Depending on the length of the entry portion, a portion of the luer may be protected by the tubular retainer. The snap point provides a snap fit for the luer within the tubular retainer.

## Description

### FIELD OF THE INVENTION

The present invention relates generally to catheters intended for deployment within a patient's vasculature, and more particularly, to a packaging assembly for a catheter.

### BACKGROUND OF THE INVENTION

Catheters may be inserted into a patient's vasculature and deployed at various locations within the patient for a wide variety of purposes and medical procedures. For example, one type of catheter is used in percutaneous catheter intervention (PCI) for the treatment of a vascular constriction generally known as a stenosis. In this instance, the catheter has a distally mounted balloon that can be placed, in a deflated or collapsed condition, within the stenosis, and then inflated or expanded to dilate the narrowed lumen of a blood vessel. This type of balloon dilation therapy is generally referred to as percutaneous transluminal angioplasty (PTA). When the treatment is more specifically intended for vessels of the heart, the process is known as percutaneous transluminal coronary angioplasty (PTCA). PTCA is utilized to open coronary arteries that have been occluded by a build up of cholesterol fats and atherosclerotic plaque. The balloon at the distal end of the catheter is inflated causing a widening at the site of the stenosis.

Dilation of an occlusion, however, can form flaps, fissures, and dissections, that may result in reclosure of the dilated vessel or even perforations in the vessel wall. Implantation of a stent can provide support for such flaps and dissections and thereby prevent reclosure of the vessel or provide a patch repair for a perforated vessel wall until corrective surgery can be performed. The stent is typically a cylindrically shaped device formed from wire(s) or a metal tube and is intended to act as a permanent prosthesis. The stent is deployed in a body lumen in a radially compressed configuration and is subsequently radially expanded to contact and support a body lumen. The stent can be implanted during an angioplasty procedure by using a balloon catheter having deployed thereon a compressed stent that has been loaded onto the balloon. The stent radially expands as the balloon is inflated thus forcing the stent into contact with the body lumen and forming a supporting relationship with the lumen walls. Alternatively, self expanding stents may be deployed with a sheath-based delivery catheter. Deployment is effected after the stent has been introduced percutaneously, transported transluminally, and positioned at a desired location by the delivery catheter. In addition to angioplasty and stenting procedures, other therapeutic procedures require the use of a delivery catheter; e.g. drug delivery devices, filters, occlusion devices, diagnostic devices, and radiation treatment.

Catheters are commonly packaged and stored in a packaging hoop that consists of coiled tubing into which the catheter is inserted. A luer fitting located at the proximal end of the catheter is provided with a distal hub that fits into an opening in the tubing thus securing the catheter in the hoop. Balloons and/or stents are commonly disposed on a distal portion of catheters. When removing the catheter from the packaging hoop, an operator may grasp the proximal end of the hoop in order to separate the luer fitting from the hoop. However, by grasping the proximal end of the hoop, the opening in the tubing may become misshapen due to pressure from the fingers of the operator. Accordingly, when the balloon and/or stent attempt to pass through the opening, one or both may become damaged. Such damage may be especially problematic with coated stents which are now popular.

Accordingly, an improved packaging arrangement is desirable that discourages grasping the packaging at a location that can potentially damage an object passing through the packaging, such as a balloon and/or stent.

### BRIEF SUMMARY OF THE INVENTION

An assembly for packaging a catheter is provided. The assembly includes flexible tubing capable of being coiled, and tubular retainer having a passageway extending therethrough. The tubular retainer includes a distal section for coupling to a proximal end of the tubing. A proximal end of the tubular retainer includes an opening for receiving a luer of the catheter assembly. A keyway is provided at a proximal end of the tubular retainer, the keyway being adapted to receive an inflation port of the luer. The keyway may include a snap point at a proximal end thereof, wherein the snap point is dimensioned such that a force is required to pass the inflation port past the snap point, thereby resulting in a snap fit. The keyway may include an entry portion beginning at the proximal end of the tubular retainer, and a nose portion disposed distal of the entry portion, with a snap point disposed therebetween. Depending on the length of the entry portion, a portion of the luer may be protected by the tubular retainer. The snap point provides a snap fit for the luer within the tubular retainer.

### BRIEF DESCRIPTION OF DRAWINGS

The foregoing and other features and advantages of the invention will be apparent from the following description of the invention as illustrated in the accompanying drawings. The accompanying drawings, which are incorporated herein and form a part of the specification, further serve to explain the principles of the invention and to enable a person skilled in the pertinent art to make and use the invention. The drawings are not to scale.

FIG. 1 is a diagrammatic drawing illustrating the deployment of a balloon catheter within a patient's vasculature.

FIG. 2 is an enlarged diagrammatic drawing of a portion of FIG. 1.

FIG. 3 illustrates a conventional catheter packaging hoop with a traditional catheter partially inserted into the hoop.

FIG. 4 illustrates the conventional catheter packaging hoop of FIG. 3 with the catheter fully inserted into the hoop.

FIG. 5 is an enlarged view of a portion of FIG. 4.

FIG. 6 illustrates a person removing a catheter from the conventional catheter packaging hoop of FIG. 4.

FIG. 7 is an isometric view illustrating a catheter inserted into a conventional catheter packaging hoop coupled to a retainer in accordance with the present invention.

FIG. 8 is an isometric view illustrating a luer having an inflation port.

FIG. 9 is a perspective view of an embodiment of a retainer of the present invention.

FIG. 10 is a perspective view of another embodiment of a retainer of the present invention.

FIG. 11 is a perspective view of another embodiment of a retainer of the present invention.

FIG. 12 is a perspective view of another embodiment of a retainer of the present invention.

FIG. 13 is a perspective view of another embodiment of a retainer of the present invention.

FIG. 14 illustrates the luer of FIG. 8 inserted into the retainer of FIG. 12.

### DETAILED DESCRIPTION OF THE INVENTION

Specific embodiments of the present invention are now described with reference to the figures, where like reference numbers indicate identical or functionally similar elements. The terms "distal" and "proximal" are used in the following description with respect to a position or direction relative to the treating clinician. "Distal" or "distally" are a position distant from or in a direction away from the clinician. "Proximal" and "proximally" are a position near or in a direction toward the clinician.

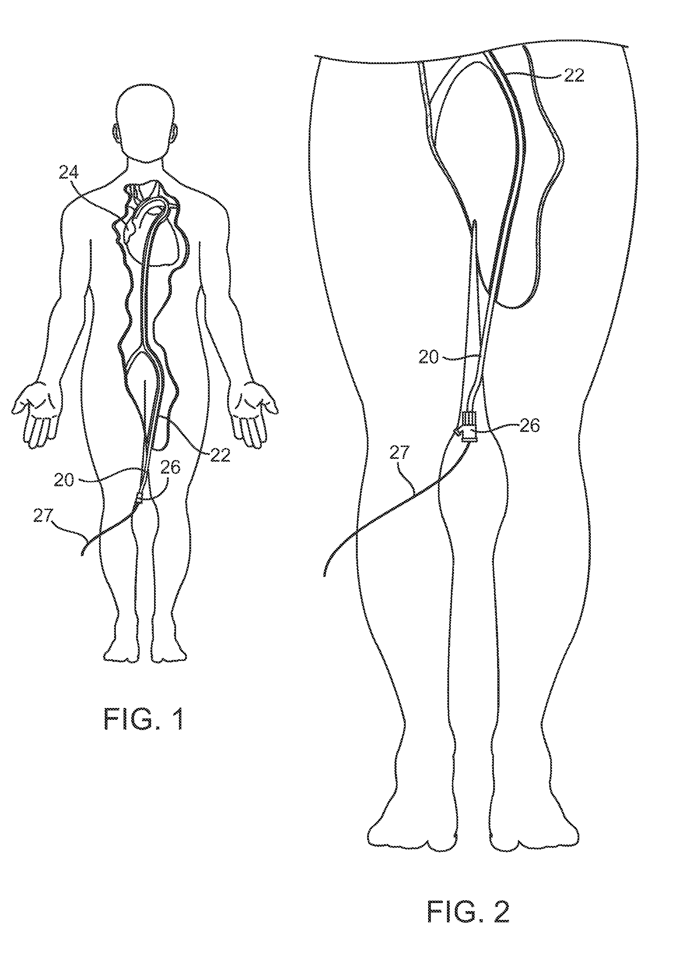

As stated previously, to treat small diameter vessels remote from an entry point into a patient, a guiding catheter may be used to span the distance. For example, referring to FIGS. 1 and 2 which illustrates the deployment of a balloon catheter within a patient's vasculature, in PTCA or stent delivery, a catheter 20 is typically inserted into a large artery 22 near the patient's groin and is then advanced towards heart 24 to the entry opening or ostium of a diseased coronary artery. A luer 26 is provided and a proximal end of catheter 20, which conventional provides a lumen through which inflation fluid can be delivered to inflate a balloon and a guidewire lumen to track the catheter to the treatment site over a guidewire, such as guidewire 27.

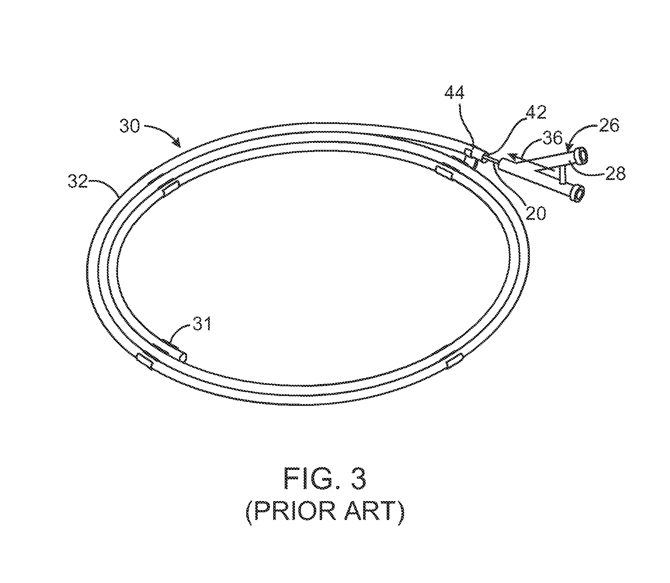

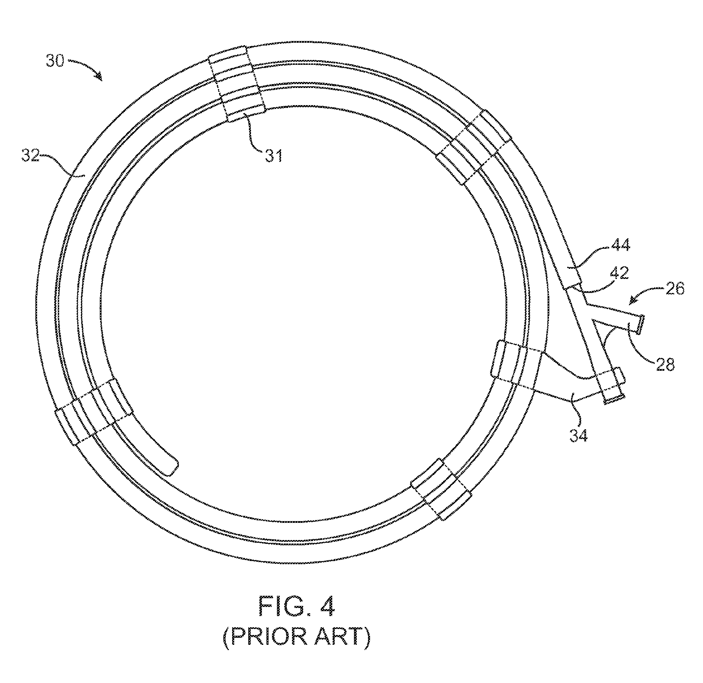

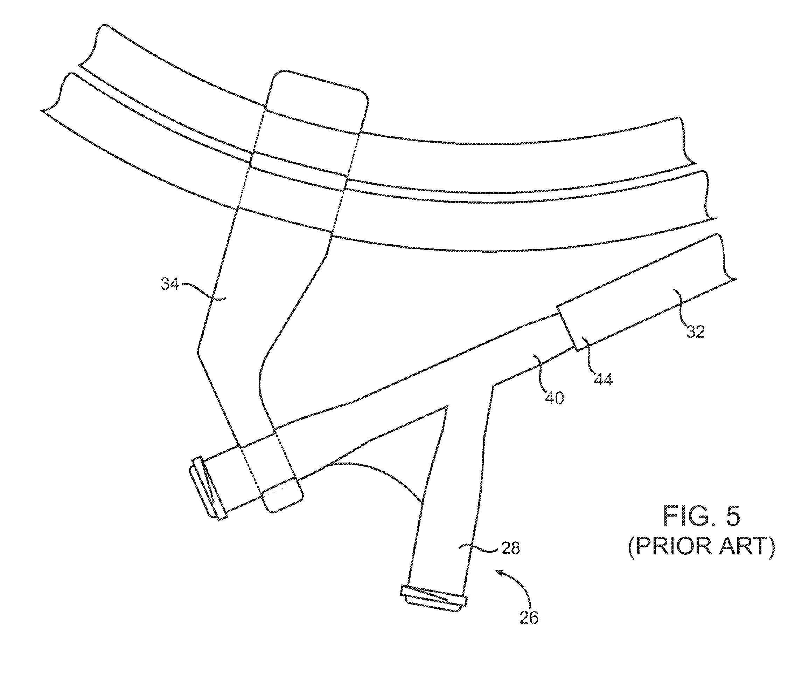

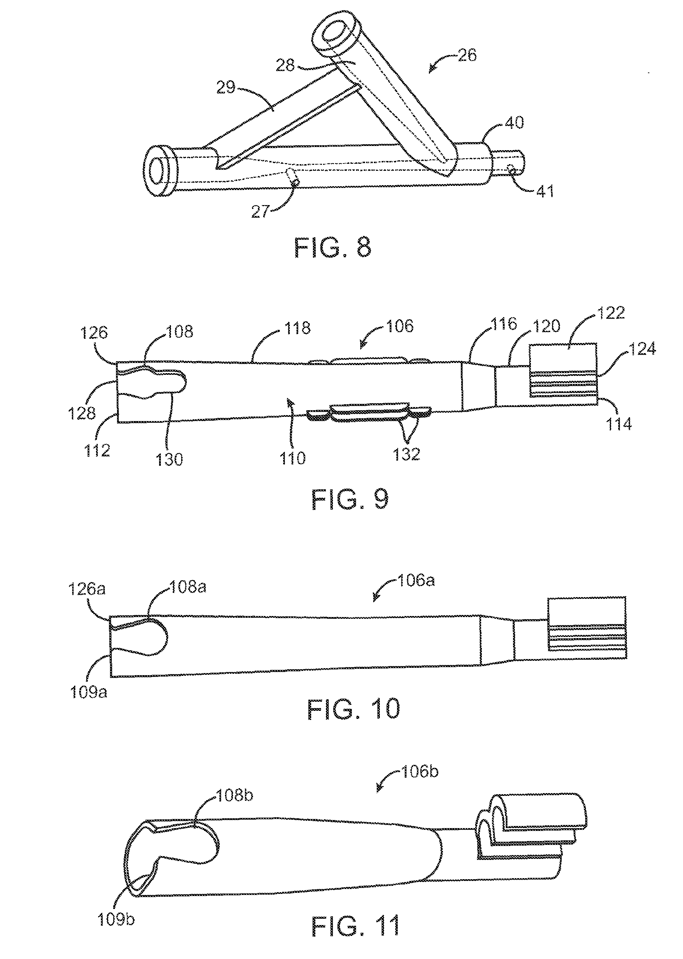

Referring now to FIGS. 3-5, catheters are commonly packaged and stored in a packaging hoop 30 as shown in FIG. 3 in accordance with the teachings of the prior art. Packaging hoop 30 consists of coils of tubing 32 into which catheter 20 is inserted in the direction indicated by arrow 36. Clips 31 are coupled to tubing 32 to maintain the tubing 32 in the hoop configuration. Luer 26, located at the proximal end of catheter 20, has a distal hub 40 that fits into opening 42 of tubing 32, thus securing catheter 20 in hoop 30 as is shown in FIGS. 4 and 5. Luer 26 includes a guidewire port 27, an inflation port 28, and a support 29, as is known in the art and shown in FIG. 8. Luer 26 also includes a distal nose 41, over which catheter 20 is disposed. As also shown in FIGS. 4 and 5, a clip 34 is coupled to tubing 32 and to luer 26 to prevent luer 26 from moving relative to hoop 30 prior to removal by an operator.

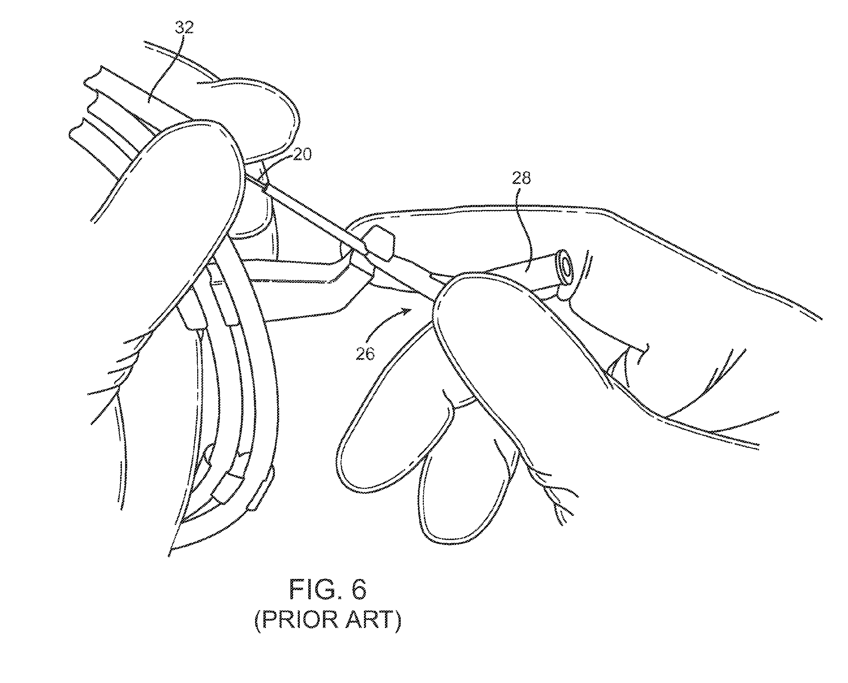

As discussed above, catheter 20 generally includes a balloon (not shown) disposed at a distal portion thereof and a stent (not shown) mounted on the balloon. As shown in FIG. 6, when removing catheter 20 from packaging hoop 30, it is common for an operator to grasp luer 26 with one hand, grasp a proximal portion 44 of tubing 32 in the other hand, and pull luer 26 away from tubing 32. However, when grasping proximal portion 44 of tubing 32, pressure may be applied to tubing 32, causing opening 42 to lose its shape. As catheter 20 continues to be removed from tubing 32, the balloon and/or stent need to pass through opening 42. However, as opening 42 may be misshapen as described above, the balloon and/or stent may be damaged upon removal.

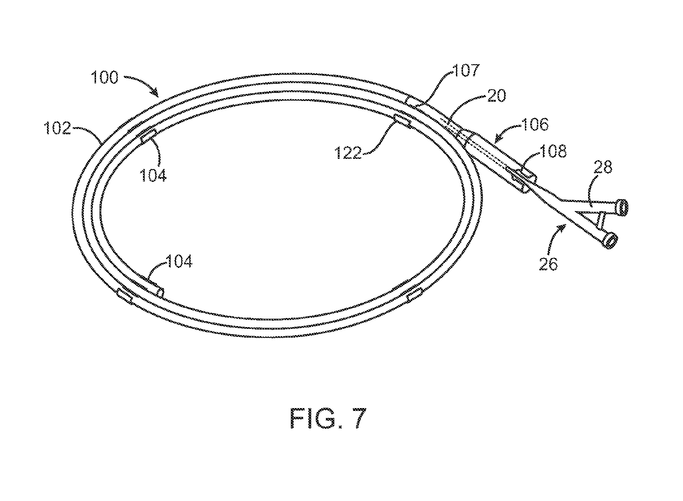

FIG. 7 shows a packaging assembly 100 in accordance with the present invention and a catheter 20 as described above. Catheter 20 includes a luer 26 as described above at a proximal end thereof. Packaging assembly 100 is similar to the packaging hoop 30 described above in that it includes tubing 102 maintained in a hoop configuration by clips 104. Additionally, a retainer 106 is disposed at a proximal end 107 of tubing 102. Retainer 106 is similar to retainer described in U.S. Published Application Publication No. 2005/0205446 A1, the contents of which are incorporated herein by reference in their entirety. However, retainer 106 includes a keyway 108 adapted to receive the inflation port 28 of luer 26. Catheter 20 is shown almost fully inserted into packaging assembly 100.

FIG. 9 shows an embodiment of retainer 106. Retainer 106 comprises a generally tubular body 110 having a proximal port 112 and a distal port 114. Tubular body 140 includes a tapered region 116 wherein the cross section of a tubular body may transition from larger diameter at a proximal region 118 to a smaller diameter at a distal region 120. The tapered region 116 may also provide a transition between a first shaped cross section, such as oval, of proximal region 118, to a second shaped cross section, such as circular, at the distal region 120, as described in U.S. Published Application Publication No. 2005/0205446 A1. Proximal port 112 of tubular body 110 is configured so as to matingly receive luer 26. Distal region of tubular body 110 is generally cylindrical and sized so as to matingly receive therein proximal end 107 of tubing 102 (FIG. 7). While press fitting proximal end 107 of tubing 102 within distal port 114 of tubular body 110 should provide sufficient force to secure retainer 106 to the tubing 102 (FIG. 7), shrink wrap may be utilized to provide added security if desired.

Retainer 106 includes a hoop coil clip 122 attached to or formed integrally with distal region 120. Hoop clip 122 is preferably formed integrally with retainer 130. Hoop clip 122 is comprised of semi-circular channels 124 which are dimensioned to matingly receive tubing 102, for example, by press-fitting. As can be seen in FIG. 7, the coils of tubing 102 are secured or held in place by retainer hoop clip 122 and additional hoop clips 104.

Keyway 108 of retainer 106 is provided at a proximal end 126 of retainer 106. Keyway 108 is essentially a portion of tubular body 110 that is removed or not formed such as to appear to be a cut-out. Keyway 108 is shaped and sized to receive inflation port 28 of luer 26. In the embodiment of FIG. 9, keyway 108 includes and entry portion 128 and a nose portion 130. Nose portion 130 is narrower that entry portion 128, thereby providing a snap-fit around inflation port 28.

The embodiment of FIG. 9 also shows protrusions 132 on an outer surface of tubular body 110. Protrusions 132 minimize contact between an operator's hand and retainer 106 as retainer 106 is gripped. Protrusions 132 also may indicate the desired location for an operator to grip retainer 106 so as to prevent or minimize distortion of any lumen through which a balloon and/or stent of catheter 20 must pass. Protrusions 132 are shown as longitudinal, but they may take any shape and orientation and still achieve the desired functionality.

FIG. 10 shows an alternative embodiment of a retainer 106a which is similar to retainer 106 of FIG. 9. However, keyway 108a of retainer 106a does not include nose portion 130 of the embodiment of FIG. 9. A snap point 109a may be included at proximal end 126a of retainer 106a. Snap point 106a is dimensioned to be slightly smaller than the remainder of keyway 108a and slightly smaller than inflation port 28, such that a small force is required to pass inflation port past snap point 109a, thereby resulting in a snap-fit. Further, retainer 106a does not include protrusions 132, although they may be included.

FIG. 11 shows another embodiment of a retainer 106b. Retainer 106b is identical to retainer 106a of FIG. 10 except that it is longitudinally shorter. Keyway 108b is identical to keyway 108a of FIG. 10.

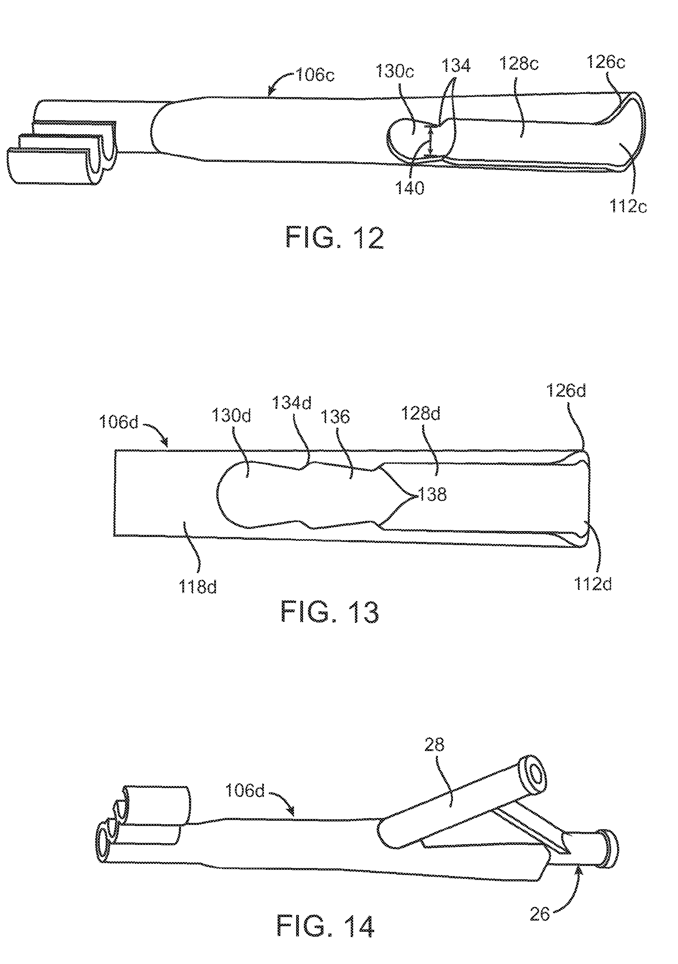

FIG. 12 shows another embodiment of a retainer 106c. Retainer 106c is similar to retainer 106 of FIG. 9, except that entry portion 128c of keyway 108c is longer than entry portion 128 of keyway 108. A snap point 134 separates entry portion 128c of keyway 108c from nose portion 130c. A longer entry portion 128c allows more of luer 26 to be disposed within retainer 106c so as to provide additional protection of luer 26, as shown in FIG. 14. Snap point 134 is dimensioned such that keyway 108c at snap point 134 is smaller than entry portion 128c and nose portion 130c, and also smaller than the diameter of inflation port 28 of luer 26. Accordingly, a force is required to pass inflation port past snap point 134, thereby resulting in a snap fit.

FIG. 13 shows another embodiment of retainer 106d. Retainer 106d is similar to retainer 106c of FIG. 12, except that a second snap point 138 is provided. Only proximal portion 118d of retainer 106d is shown in FIG. 13 for convenience. Keyway 108d of retainer 106d includes an entry portion 128d beginning at proximal end 126d. First snap point 138 separates entry portion 128d from an intermediate portion 136 of keyway 108d. Second snap point 134d separates intermediate portion 136 of keyway 108d from nose portion 130d of keyway 108d.

Dimensions for the various features of the keyways 108 described above, such as the entry portions, nose portions, and snap points, may be determined by those of ordinary skill in the art based on the size of luer 26, and inflation port 29 thereof, and factors such as the desired force to insert the luer into the keyway, fit to retain the luer within the keyway, and the force required to remove the luer from the keyway. Provided for example only, when using an Endeavor^{™} bifurcate luer, a keyway 108c as shown in FIG. 12 may have a dimension 140 at snap point 134 of approximately 4.00 mm to 5.50 mm. Preferably, such a dimension is 4.50 mm to 5.00 mm. However, it would be apparent to persons skilled in the relevant art that these dimensions would change depending on various factors, and optimal dimensions can be obtained by routine experimentation.

The retainer described herein may be made of polypropylene or polyethylene (high or low density) or any other material suitable for use as packaging for a catheter, as would be apparent to persons skilled in the relevant art.

While several embodiments of the present invention have been described above, it should be understood that they have been presented by way of illustration and example only, and not limitation. It will be apparent to persons skilled in the relevant art that various changes in form and detail can be made therein without departing from the spirit and scope of the invention. Further, it will be apparent to persons skilled in the relevant art that different features of the various embodiments may be combined with features of other embodiments without departing from the spirit and scope of the invention. Thus, the breadth and scope of the present invention should not be limited by any of the above-described exemplary embodiments, but should be defined only in accordance with the appended claims and their equivalents. It will also be understood that each feature of each embodiment discussed herein, and of each reference cited herein, can be used in combination with the features of any other embodiment. All patents and publications discussed herein are incorporated by reference herein in their entirety.

## Claims

1. An assembly for packaging a catheter having a flexible distal shaft and a proximal fitting coupled thereto, the assembly comprising:
a flexible tube capable of being coiled and having a proximal end;
a tubular retainer having a passageway extending therethrough and having a proximal end and a distal section, said distal section for matingly receiving the proximal end of said flexible tube, said proximal end including an opening for receiving the proximal fitting of the catheter, wherein said tubular retainer includes a keyway adapted to receive an inflation port of the proximal fitting of the catheter.

2. The assembly of claim 1, wherein said keyway includes a snap point disposed at the proximal end of said tubular retainer, wherein said snap point is dimensioned to be smaller than the inflation port of the proximal fitting such that a force is required to pass the inflation port past said snap point and into the keyway.

3. The assembly of claim 1 or claim 2, wherein said keyway includes an entry portion beginning at the proximal end said tubular retainer, a nose portion disposed distal of said entry portion, and a snap point disposed between the entry portion and the nose portion, wherein said snap point is dimensioned to be smaller than the inflation port of the proximal fitting such that a force is required to pass the inflation port past said snap point and into said nose portion.

4. The assembly of any preceding claim, wherein said keyway includes an entry portion beginning at the proximal end said tubular retainer, a nose portion disposed distal of said entry portion, an intermediate portion disposed between said entry portion and said nose portion, a first snap point disposed between said entry portion and said intermediate portion, and a second snap point disposed between said intermediate portion and said nose portion, wherein said first and second snap points are dimensioned to be smaller than the inflation port of the proximal fitting such that a force is required to pass the inflation port past each of said snap points.

5. The assembly of any preceding claim, wherein said tubular retainer includes projections on an outer surface thereof.

6. The assembly of any preceding claim, wherein said distal section has a substantially circular cross section.

7. The assembly of any preceding claim, wherein said tubular retainer further includes a clip fixedly coupled to said distal section for securing a portion of said flexible tube.

8. The assembly of any preceding claim, wherein said clip comprises at least one substantially semi-cylindrical groove for receiving a portion of said flexible tube therein.

9. A catheter packing device comprising:
a tubular body having a passageway extending therethrough, a proximal end, a distal section, and a keyway beginning at the proximal end, wherein said keyway is adapted to receive a fitting of a catheter.

10. The device of any preceding claim, wherein said keyway includes a snap point disposed at the proximal end of said tubular body, wherein said snap point is dimensioned to be smaller than a portion of the fitting of the catheter such that a force is required to pass the portion past said snap point and into said keyway.

11. The device of any preceding claim, wherein said keyway includes an entry portion beginning at the proximal end said tubular body, a nose portion disposed distal of said entry portion, and a snap point disposed between the entry portion and the nose portion, wherein said snap point is dimensioned to be smaller than entry portion and the nose portion.

12. The device of any preceding claim, wherein said keyway includes an entry portion beginning at the proximal end said tubular body, a nose portion disposed distal of said entry portion, an intermediate portion disposed between said entry portion and said nose portion, a first snap point disposed between said entry portion and said intermediate portion, and a second snap point disposed between said intermediate portion and said nose portion, wherein said first and second snap points are dimensioned to be smaller than entry portion, the intermediate portion, and the nose portion.

13. The device of any preceding claim, further comprising a protrusion extending from an outer surface of said tubular body.

14. The device of any preceding claim, wherein said distal section is configured for coupling to a coiled catheter packaging hoop.

15. The device of any preceding claim, further comprising a clip fixedly coupled to said distal section for securing a coil of the packaging hoop.

16. The device of any preceding claim, wherein said clip comprises at least one substantially semi-cylindrical groove for receiving a coil of the packaging hoop therein.
